(19) Europäisches Patentamt
European Patent Office
Office européen des brevets

(11) **EP 4 232 181 B1**

(12) **EUROPÄISCHE PATENTSCHRIFT**

(45) Veröffentlichungstag und Bekanntmachung des Hinweises auf die Patenterteilung:
**06.05.2026   Patentblatt 2026/19**

(21) Anmeldenummer: **21831224.7**

(22) Anmeldetag: **30.11.2021**

(51) Internationale Patentklassifikation (IPC):
***B01D 53/04*** *(2006.01)*

(52) Gemeinsame Patentklassifikation (CPC):
**B01D 53/0438; B01D 53/0446; B01D 53/0462;**
B01D 2253/202; B01D 2257/504; B01D 2258/06;
B01D 2259/4566; Y02C 20/40

(86) Internationale Anmeldenummer:
**PCT/EP2021/083561**

(87) Internationale Veröffentlichungsnummer:
**WO 2022/122473 (16.06.2022 Gazette 2022/24)**

(54) **CO-ABSORBER**

COABSORBER

ÉLECTROLYSEUR À CO

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.12.2020   DE 102020215686**

(43) Veröffentlichungstag der Anmeldung:
**30.08.2023   Patentblatt 2023/35**

(73) Patentinhaber:
• **TKMS GmbH**
  **24143 Kiel (DE)**
• **thyssenkrupp AG**
  **45143 Essen (DE)**

(72) Erfinder:
• **BÜCHNER, Richard**
  **23611 Bad Schwartau (DE)**
• **KUHLENCORD, Moritz**
  **22529 Hamburg (DE)**

(74) Vertreter: **thyssenkrupp Intellectual Property GmbH**
**ThyssenKrupp Allee 1**
**45143 Essen (DE)**

(56) Entgegenhaltungen:
EP-A1- 2 963 107          WO-A2-2014/011081
DE-A1- 102018 212 898     DE-B4- 102008 015 150
US-A- 5 762 692

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen regenerativen $CO_2$-Absorber, insbesondere zur Verwendung an Bord eines Unterseebootes.

An Bord eines Unterseebootes muss die Atemluft ständig erneut werden, das von Mannschaft produzierte $CO_2$ muss entfernt und der verbrauchte Sauerstoff wieder ergänzt werden. Da dieses für die Besatzung lebenswichtig ist, muss eine kontinuierliche Aufbereitung der Atemluft zuverlässig und auch über lange Zeiträume sichergestellt sein.

Zur Abtrennung von Kohlenstoffdioxid gibt es eine Reihe von Methoden. Insbesondere werden Filter aus Atemkalk oder Lithiumhydroxid verwendet. Nachteil dieser Filter ist jedoch, dass diese nicht während der Fahrt regeneriert werden können. Somit muss das Unterseeboot ausreichend Filterkapazität mitführen. Dieses führt zu einer hohen Gewichtsbelastung. Zusätzlich müssen die Filter regelmäßig getauscht werden, wobei das Risiko aus dem Umgang mit den Stoffen besteht, weil sowohl bei Atemkalk als auch Lithiumhydroxid in Verbindung mit Wasser eine mehr oder weniger starke Lauge gebildet wird.

Zur Abtrennung von Kohlenstoffdioxid wird als Filter, welcher regeneriert werden kann, teilweise ein Feststoff mit funktionellen Amingruppen eingesetzt. Derartige Filter werden zunächst mit Kohlenstoffdioxid aus Atemluft beladen und anschließend in einem Regenerationszyklus durch Erwärmen regeneriert. Nachteil ist, dass das Amin beim Erwärmen degradiert.

Aus der DE 10 2008 015 150 B4 ist ein Unterseeboot mit einer Belüftungseinrichtung und einer $CO_2$-Absorptionseinrichtung bekannt.

Aus der DE 10 2018 212 898 A1 ist ein $CO_2$-Absorber mit einer Wasserinjektion zur Kühlung bekannt. Die hohe Beladbarkeit des Amins mit Wasser hat sich dabei jedoch als weniger vorteilhaft herausgestellt.

Aus der DE 697 28 061 T2 ist ein Reinigungsgerät auf dem Gebiet der Narkose bekannt. Aus der WO 2017/212 381 A1 ist die Bindung von $CO_2$ aus Abgasen bekannt.

Aus der WO 2014 011 081 A2 ist eine Vorrichtung und ein Verfahren zum Trocknen von Gasen bekannt.

Aus der EP 2 963 107 A1 ist ein Verfahren zum Raffinieren von Biomethan bekannt. Aus der US 5 762 692 A Ist eine Emissionsschutzvorrichtung für Automobile bekannt. Aus der DE 10 2008 015 150 B4 ein Unterseeboot mit einer Belüftungsvorrichtung bekannt.

Aufgabe der Erfindung ist es, eine Vorrichtung zu schaffen, welche für einen langzeitstabilen Einsatz insbesondere in einem Unterseeboot geeignet ist, also zuverlässig $CO_2$ absorbiert und in beengter räumlicher Umgebung leicht die notwendigen Wartungsschritte erlaubt.

Gelöst wird diese Aufgabe durch die Vorrichtung mit den in Anspruch 1 angegebenen Merkmalen.

Vorteilhafte Weiterbildungen ergeben sich aus den Unteransprüchen, der nachfolgenden Beschreibung sowie den Zeichnungen.

Die erfindungsgemäße Vorrichtung dient zur Abtrennung von Kohlenstoffdioxid aus einem Gasgemisch, insbesondere zur Abtrennung von Kohlendioxid aus der Atemluft an Bord eines Unterseebootes. Die Vorrichtung weist einen Feststoff zur Aufnahme von Kohlenstoffdioxid auf. Bei dem Feststoff handelt es sich bevorzugt um ein Polymer mit funktionellen Amingruppen. Die Vorrichtung weist eine Wärmetauschvorrichtung im Feststoff auf. Die Wärmetauschvorrichtung dient dazu den Feststoff zur Desorption des Kohlendioxids zu erwärmen und nach der Desorption wieder abzukühlen. Zusätzlich kann der Feststoff auch während der Absorption des Kohlendioxids gekühlt werden, um eine ungewollte Erwärmung durch die exotherme Reaktion zu verhindern und so die Absorption zu optimieren. Die Vorrichtung weist eine Hülle auf, wobei die Hülle evakuierbar ist. Erfindungsgemäß wird auch ein Druck von 10 kPa oder kleiner als Vakuum im Sinne der Erfindung angesehen, da dadurch sowohl Sauerstoff ausreichend von dem Feststoff vor dem Erwärmen entfernt werden kann, als auch die Desorption von Kohlendioxid ausreichend unterstützt wird. Die Hülle muss entsprechend stabil ausgebildet sein, um einen solchen Druck standhalten zu können, wobei zusätzlich zu berücksichtigen ist, dass der Druck im Inneren eines Unterseebootes stark schwanken kann und auch Werte bis 160 kPa erreichen kann. Die Vorrichtung weist einen Einlass in der Hülle für das Gasgemisch auf, wobei der Einlass oberhalb des Feststoffs angeordnet ist. Hierdurch wird der Feststoff von oben nach unten durchströmt.

Erfindungsgemäß liegt der Feststoff zur Aufnahme von Kohlendioxid partikulär vor. Partikulär ist im Sinne der Erfindung beispielsweise kugelförmig. Die Größe der Partikel der Feststoffe liegt vorzugsweise im Bereich von 0,1 mm bis 50 mm, bevorzugt von 1 mm bis 10 mm. Durch die Verwendung eines partikulären Feststoffes ist der Austausch auch in einem räumlich beengten Unterseeboot deutlich erleichtert und auch die Lagerung des Feststoffes ist einfacher gegenüber beispielsweise scheibenförmigen Feststoffen.

Die Hülle weist ganz besonders bevorzugt einen runden Querschnitt auf. Der Einlass ist zur Erzeugung einer radialen Strömung des Gasgemisches oberhalb des Feststoffes ausgebildet. Beispielsweise und bevorzugt ist dieser nicht zentral, sondern seitlich versetzt angeordnet, wobei die Mittelachse des Einlassrohres nicht auf die Mitte der Vorrichtung gerichtet ist und vorzugsweise horizontal verläuft oder parallel zur einlassseitigen Oberfläche, also oberen Ebene, des partikulären Feststoffs ausgerichtet ist. Dadurch ist die Strömungsrichtung des Einlasses nicht auf die Mitte der Vorrichtung gerichtet. Vorzugsweise verläuft die Strömungsrichtung des Einlasses horizontal und weist also keine vertikale Komponente auf, welche auf den partikulären Feststoff gerichtet ist. Hierdurch wird die kinetische Energie zur Erzeugung einer radialen Strömung genutzt. Insbesondere weist der Einlass einen deutlich geringeren Querschnitt als die Hülle im horizontalen (bevorzugt runden) Querschnitt. Dieses bewirkt, dass das Gasgemisch mit höherer Geschwindigkeit einströmt, dadurch eine radiale Strömung in der Hülle erzeugt, die vorzugsweise größtenteils parallel zur Oberfläche des Feststoffes verläuft. Die anschließende Strömung des Gasgemisches durch den Feststoff von oben nach unten erfolgt dann mit entsprechend geringerer senkrechter Strömungsgeschwindigkeitskomponente (durch den deutlich größeren Querschnitt bei gleichem Volumenstrom). Dieses hat zwei positive Effekte. Zum einen wird dadurch eine gleichmäßige und den partikulären Feststoff nicht oder nur geringfügig aufwirbelnde Strömung erzeugt. Zum anderen erhöht die geringere senkrechte Strömungsgeschwindigkeitskomponente die Kontaktzeit zum Feststoff und damit die Absorption von Kohlendioxid aus dem Gasgemisch.

Erfindungsgemäß sind in der Hülle und auf Höhe des Einlasses Leitelemente zur Verstärkung einer radialen Strömung angeordnet. Hierdurch kann die radiale Strömung weiter optimiert und eine möglichst gleichmäßige Anströmung des Feststoffes erreicht werden. Insbesondere sind die Leitelemente in Form senkrecht angeordneter Leitbleche ausgeführt, welche am oberen Ende der Hülle im Inneren angeordnet sind. Besonders bevorzugt weisen die Leitelemente eine geschwungene Form auf, um die radiale Strömung zu fördern. Weiter bevorzugt sind die Position und Ausrichtung des Einlasses und die Position und Form der Leitelemente für eine optimale radiale Strömung aufeinander abgestimmt. Beispielsweise sind die Leitbleche koaxial zu einer gedachten Achse in Form eines Kreisabschnitts geformt, der einen Kreisabschnitt von 45° bis 180° bilden kann und können eine Höhe aufweisen, die zumindest dem Durchmesser des Einlassrohrs entspricht. Sie können dabei direkt hinter dem Einlassrohr angeordnet sein, sodass das einströmende Gas direkt durch die Leitelemente geführt wird.

Bevorzugt ist die radialen Strömung des Gasgemisches eine laminare Strömung und keine turbulente Strömung. Diese kann neben der Größe des Einlassrohres und damit die Strömungsgeschwindigkeit des Gases auch beispielsweise durch die Leitelemente unterstützt werden. Je geringer der turbulente Anteil der Strömung ist, umso geringer ist die Gefahr der Verwirbelung des Feststoffes.

In einer weiteren Ausführungsform der Erfindung ist der Feststoff in einer horizontal angeordneten Absorptionsschicht angeordnet ist, wobei die Absorptionsschicht an der Oberseite ein oberes Begrenzungselement und an der Unterseite ein unteres Begrenzungselement aufweist. Das obere Begrenzungselement und das untere Begrenzungselement werden jeweils durch ein Gitter oder Lochblech gebildet, also durch etwas, durch das das Gasgemisch leicht durchströmen kann, was aber den Feststoff sicher zurückhält. Daher sind die Öffnungen des Gitters oder des Lochblechs kleiner als die Partikelgröße des Feststoffes. Besonders bevorzugt ist die Absorptionsschicht scheibenförmig, die Dicke ist somit geringer als der Durchmesser. Der Feststoff liegt in der Absorptionsschicht als eine Schüttung vor, was einen Füllgrad in der Größenordnung von $2/3$ nahelegt. Entsprechend ist der Strömungswiderstand des Feststoffes groß. Daher ist es vorteilhaft, eine große Fläche zu durchströmen und nur eine geringe Schichtdicke des Feststoffes, da dieses beides den Strömungswiderstand des Feststoffes insgesamt reduziert und so den Energiebedarf für das Fördern des Gasgemisches durch die Vorrichtung minimiert. Insbesondere erstreckt sich die Absorptionsschicht über den gesamten Strömungsquerschnitt, so dass die Strömung den Feststoff durchströmen muss. Die zu durchströmende Dicke der Absorptionsschicht ist dabei über den gesamten Strömungsquerschnitt im Wesentlichen konstant. Die Begrenzungselemente können auch mehrlagig ausgebildet sein, beispielsweise aus einem ersten groben Gitter mit sehr großen Öffnungen (größer als der Feststoff), dafür aber mit sehr stabilen Trageelementen. Auf diesem ersten groben Gitter wird dann beispielsweise ein feinmaschiges Feingitter angeordnet, dass den Feststoff zurückhält und selber durch das erste grobe Gitter getragen und gestützt wird. Hierdurch wird die optimale Tragkraft des ersten groben Gitters mit der optimalen Rückhalteeigenschaft des zweiten Feingitters kombiniert.

In einer weiteren Ausführungsform der Erfindung weist das obere Begrenzungselement vertikale Strukturen auf, wobei sich die vertikalen Strukturen in den Feststoff erstrecken. Diese vertikalen Strukturen dienen dazu, ein Verrutschen des Feststoffes, beispielsweise beim Auftauchen, beim Abtauchen oder bei Krängung zu verhindern, insbesondere, wenn sich oben ein Ausgleichsvolumen zur Aufnahme von Volumenänderung befindet. Dabei können die vertikalen Strukturen beispielsweise durch senkrecht nach unten angeordnete Elemente gebildet werden, die beispielsweise die gleiche Struktur wie das Begrenzungselement selber aufweisen können, beispielsweise auch aus einem Gitter bestehen. In einem alternativen Beispiel ist das obere Begrenzungselement selber gewellt ausgeführt und weist damit selber Erhöhungen und Vertiefungen auf. Da der Feststoff von oben angeströmt wird, wird dieser nicht fluidisiert, wie dieses bei einer Anströmung von unten der Fall sein könnte. Daher reichen diese vertikalen Strukturen zur Stabilisierung aus.

In einer weiteren Ausführungsform der Erfindung weist die Vorrichtung eine Pumpe zum Evakuieren der Hülle auf, wobei die Pumpe vorzugsweise eine Wasserringpumpe ist. Die Wasserringpumpe hat zwei wesentliche Vorteile. Zum einen wird durch den Flüssigkeitsspalt in der Pumpe die direkte Berührung vermieden, sodass diese Pumpe vergleichsweise leise ist, was die akustische Signatur des Unterseebootes reduziert.

Zum anderen kondensiert Wasser direkt in der Pumpe aus und wird mit dem Wasser der Pumpe aus dem System entfernt. Dieses ist besonders vorteilhaft, da der Feststoff neben der Bindung von Kohlendioxid auch zur Aufnahme von Wasser aus der Atemluft neigt und somit neben $CO_2$ auch Wasser bei der Desorption in größeren Mengen abgegeben wird. Durch das Auskondensieren wird das Wasser entfernt und das zu fördernde Gasvolumen dadurch gleichzeitig reduziert. Gegenüber einem vor einer anderen Pumpe angeordneten Wasserabscheider wird zusätzlich noch Energie und Bauplatz gespart.

In einer weiteren Ausführungsform der Erfindung weist die Vorrichtung eine pneumatische Fördervorrichtung für den Feststoff auf. Insbesondere weist die pneumatische Fördervorrichtung ein Anschlussrohr auf. Das Anschlussrohr ist zur Entnahme des Feststoffes aus der Hülle evakuierbar. Weiter ist der Feststoff mit einem Gasstrom durch das Anschlussrohr in die Hülle einbringbar.

Um das Ausbringen des Feststoffes zu fördern, kann beispielsweise dadurch unterstützt werden, dass Gas durch den Einlass Gas zugeführt wird, während durch das Anschlussrohr evakuiert wird. Dadurch wird ein fördernder Gasstrom erzielt. Ebenso kann beim Einbringen gleichzeitig zum Fördern mit einem Gasstrom ein abpumpen (evakuieren) an der Hülle erfolgen, wie beispielsweise während der Desorption. Auch hierdurch kann ein kontinuierlicher Gasstrom für den Eintrag des Feststoffes erreicht werden.

In einer weiteren Ausführungsform der Erfindung weist die Wärmetauschvorrichtung ein Fluid als Wärmeträger auf. Beispielsweise und bevorzugt ist die Wärmetauschvorrichtung lamellenartig ausgeführt. Besonders bevorzugt sind die Lamellen der Wärmetauschvorrichtung parallel zur Strömungsrichtung angeordnet. Vor dem Umschalten einer Vorrichtung zur Abtrennung von Kohlenstoffdioxid vom Absorptions- in den Desorptionsbetrieb kann zur Energieeinsparung das Wärmeträgerfluid zwischen zwei Vorrichtungen zur Abtrennung von Kohlenstoffdioxid bis zum Temperaturausgleich zirkulieren.

In einer weiteren Ausführungsform der Erfindung weist die Vorrichtung einen Kühler auf, wobei der Kühler vor dem Einlass angeordnet ist. Hierdurch wird das eintretende Gas abgekühlt, wodurch mehr $CO_2$ absorbiert werden kann. Besonders bevorzugt ist der Kühler in Form eines Wärmetauschers ausgebildet, wobei der Wärmetauscher derart mit der Vorrichtung verbunden ist, dass das Gas erst durch den Wärmetäuscher fließt, von dort in die Vorrichtung strömt und anschließend zurück in den Wärmetäuscher geführt wird, derart, dass der Wärmetäuscher das in den Einlass einströmende Gas abkühlt und im Gegenzug das aus dem Auslass austretende Gas erwärmt. Dadurch wird der Energiebedarf minimiert und die Temperatur der Umgebung nicht beeinflusst.

In einer weiteren Ausführungsform der Erfindung weist der Kühler einen Wasserablass auf. Hierdurch kann im Kühler kondensiertes Wasser entfernt werden, was zu einer geringeren Wasseraufnahme des Feststoffs zur Aufnahme von Kohlenstoffdioxid führt.

Nachfolgend ist die erfindungsgemäße Vorrichtung anhand eines in den Zeichnungen dargestellten Ausführungsbeispiels näher erläutert.

Fig. 1    Querschnitt durch die erste Vorrichtung

Fig. 2    Querschnitt durch die zweite Vorrichtung

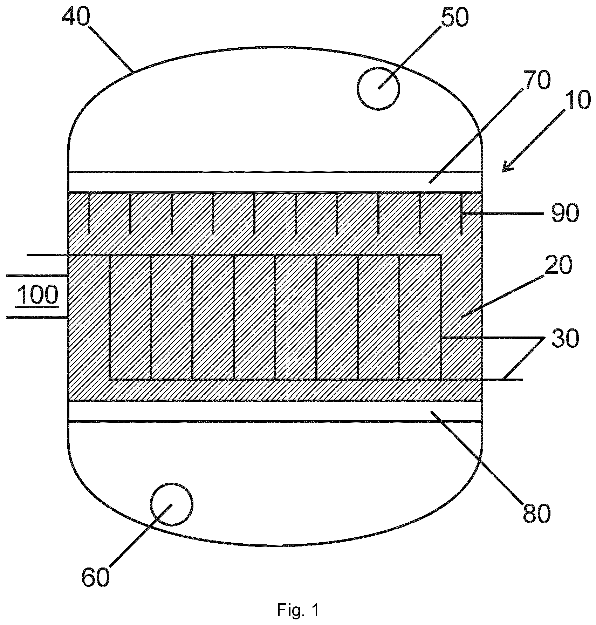

In Fig. 1 ist ein Querschnitt durch die erste Vorrichtung 10 zu sehen. Innerhalb einer Hülle 40 ist ein Feststoff 20 angeordnet, beispielsweise ein Polymer mit funktionellen Amingruppen in Form von Kügelchen mit einem Durchmesser von circa 1 mm. Im Regelbetrieb wird durch den Einlass 50 die Umgebungsluft des Unterseebootes eingeführt, im Feststoff 20 wird $CO_2$ gebunden und das von $CO_2$ abgereicherte Gasgemisch durch den Auslass 60, gegebenenfalls über einen nachgelagerten Ammoniakfilter, wieder in die Umgebungsluft abgegeben. Der partikuläre Feststoff wird durch ein oberes Begrenzungselement 70 und ein unteres Begrenzungselement 80 in der Absorptionsschicht zwischen diesen beiden gehalten. Dazu sind das obere Begrenzungselement 70 und das untere Begrenzungselement 80 in Form von Gittern mit einer Maschenweite von circa 0,5 mm und Drahtstärke von circa 0,02 mm ausgeführt. Eine Wärmetauschvorrichtung 30 ist lamellenartig in der Absorptionsschicht angeordnet und damit im Kontakt mit dem Feststoff 20. Hierdurch kann der Feststoff während des Regelbetriebs bei der Absorption des $CO_2$ gekühlt werden, während der Desorption während der Regeneration erwärmt werden sowie nach der Desorption und vor der erneuten Zuführung von zu reinigender Luft ebenfalls abgekühlt werden. Dazu wird die Wärmetauschvorrichtung 30 je nach Anwendungsfall beispielsweise und bevorzugt mit warmen beziehungsweise kaltem Wasser durchflossen. Um höhere Temperaturen, beispielsweise von 150 °C zu realisieren, ist die Wärmetauschvorrichtung 30 entsprechend für hohe Drücke des Wassers ausgelegt.

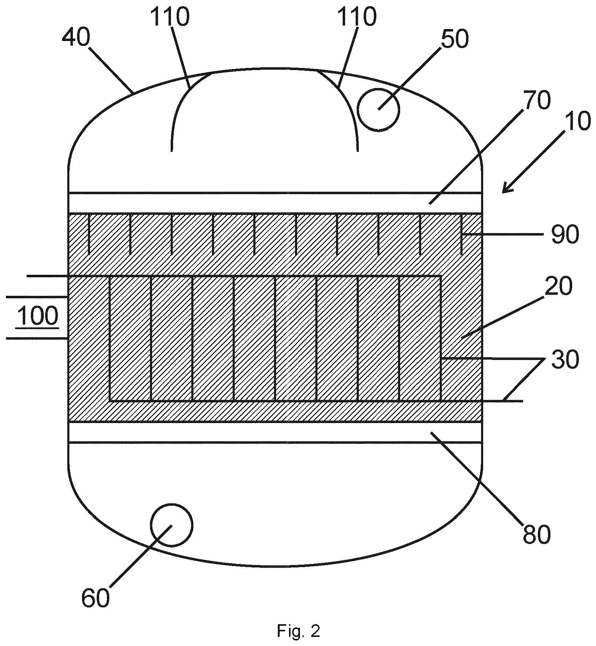

Die in Fig. 2 gezeigte zweite Vorrichtung 10 weist zusätzlich zu den anderen Merkmalen der in Fig. 1 gezeigten ersten Vorrichtung 10 zusätzliche Leitelemente 110 auf, um den durch den asymmetrisch angeordneten Einlass 50 eintretenden Gasstrom zusätzlich in Rotation zu versetzen.

Bezugszeichen

10    Vorrichtung zur Abtrennung von Kohlenstoffdioxid
20    Feststoff
30    Wärmetauschvorrichtung
40    Hülle

50 Einlass
60 Auslass
70 oberes Begrenzungselement
80 unteres Begrenzungselement
90 vertikale Strukturen
100 pneumatische Fördervorrichtung
110 Leitelement

**Patentansprüche**

1. Vorrichtung (10) zur Abtrennung von Kohlenstoffdioxid aus einem Gasgemisch, wobei die Vorrichtung (10) einen Feststoff (20) zur Aufnahme von Kohlenstoffdioxid aufweist, wobei die Vorrichtung (10) eine Wärmetauschvorrichtung (30) des Feststoffs (20) aufweist, wobei die Vorrichtung (10) eine Hülle (40) aufweist, wobei die Hülle (40) evakuierbar ist, wobei die Vorrichtung (10) einen Einlass (50) in der Hülle (40) für das Gasgemisch aufweist, wobei der Einlass (50) oberhalb des Feststoffs (20) angeordnet ist, **dadurch gekennzeichnet, dass** der Feststoff (20) zur Aufnahme von Kohlendioxid partikulär vorliegt, wobei der Einlass (50) zur Erzeugung einer radialen Strömung des Gasgemisches oberhalb des Feststoffes (20) ausgebildet ist, wobei in der Hülle (40) und auf Höhe des Einlasses (50) Leitelemente (110) zur Verstärkung einer radialen Strömung angeordnet sind.

2. Vorrichtung (10) nach Anspruch 1, **dadurch gekennzeichnet, dass** die Hülle (40) einen runden Querschnitt aufweist.

3. Vorrichtung (10) nach einem der vorstehenden Ansprüche, **dadurch gekennzeichnet, dass** der Feststoff (20) in einer horizontal angeordneten Absorptionsschicht angeordnet ist, wobei die Absorptionsschicht an der Oberseite ein oberes Begrenzungselement (70) und an der Unterseite ein unteres Begrenzungselement (80) aufweist, wobei das obere Begrenzungselement (70) und das untere Begrenzungselement (80) jeweils durch ein Gitter oder Lochblech gebildet werden, wobei die Öffnungen des Gitters oder des Lochblechs kleiner sind als die Partikelgröße des Feststoffes (20).

4. Vorrichtung (10) nach Anspruch 3, **dadurch gekennzeichnet, dass** das obere Begrenzungselement (70) vertikale Strukturen (90) aufweist, wobei sich die vertikalen Strukturen in den Feststoff (20) erstrecken.

5. Vorrichtung (10) nach einem der vorstehenden Ansprüche, **dadurch gekennzeichnet, dass** die Vorrichtung (10) eine Pumpe zum Evakuieren der Hülle (40) aufweist, wobei die Pumpe eine Wasserringpumpe ist.

6. Vorrichtung (10) nach einem der vorstehenden Ansprüche, **dadurch gekennzeichnet, dass** die Vorrichtung (10) eine pneumatische Fördervorrichtung (100) für den Feststoff (20) aufweist.

7. Vorrichtung (10) nach Anspruch 6, **dadurch gekennzeichnet, dass** die pneumatische Fördervorrichtung ein Anschlussrohr aufweist, wobei das Anschlussrohr zur Entnahme des Feststoffes (20) aus der Hülle (40) evakuierbar ist, wobei der Feststoff (20) mit einem Gasstrom durch das Anschlussrohr in die Hülle (40) einbringbar ist.

8. Vorrichtung (10) nach einem der vorstehenden Ansprüche, **dadurch gekennzeichnet, dass** die Wärmetauschvorrichtung (30) ein Fluid als Wärmeträger aufweist.

9. Vorrichtung (10) nach einem der vorstehenden Ansprüche, **dadurch gekennzeichnet, dass** die Vorrichtung (10) einen Kühler aufweist, wobei der Kühler vor dem Einlass (50) angeordnet ist.

10. Vorrichtung (10) nach Anspruch 9, **dadurch gekennzeichnet, dass** der Kühler einen Wasserablass aufweist.

**Claims**

1. An apparatus (10) for separating carbon dioxide from a gas mixture, wherein the apparatus (10) includes a solid (20) for absorption of carbon dioxide, wherein the apparatus (10) includes a heat exchange apparatus (30) for the solid (20), wherein the apparatus (10) includes a shell (40), wherein the shell (40) is evacuable, wherein the apparatus (10) has an inlet (50) in the shell (40) for the gas mixture, wherein the inlet (50) is disposed above the solid (20), **characterized in that** the solid (20) for absorption of carbon dioxide is in particulate form, wherein the inlet (50) is designed to generate a radial flow of the gas mixture above the solid (20), wherein conductive elements (110) are arranged in the shell (40) and at the level of the inlet (50).

2. The apparatus (10) as claimed in claim 1, **characterized in that** the shell (40) has a round cross section.

3. The apparatus (10) as claimed in any of the preceding claims, **characterized in that** the solid (20) is disposed in an absorption layer in a horizontal arrangement, wherein the absorption layer has an upper boundary element (70) at the top end and a lower boundary element (80) at the bottom end, wherein the upper boundary element (70) and the lower boundary element (80) are each formed by a grid or perforated plate, wherein the openings of the grid or of the perforated plate are smaller than the particle size of the solid (20).

4. The apparatus (10) as claimed in claim 3, **characterized in that** the upper boundary element (70) has vertical structures (90), wherein the vertical structures extend into the solid (20).

5. The apparatus (10) as claimed in any of the preceding claims, **characterized in that** the apparatus (10) has a pump for evacuating the shell (40), wherein the pump is a water-ring pump.

6. The apparatus (10) as claimed in any of the preceding claims, **characterized in that** the apparatus (10) has a pneumatic conveying apparatus (100) for the solid (20).

7. The apparatus (10) as claimed in claim 6, **characterized in that** the pneumatic conveying apparatus has a connecting tube, wherein the connecting tube is evacuable for removal of the solid (20) from the shell (40), wherein the solid (20) can be introduced into the shell (40) with a gas stream through the connecting tube.

8. The apparatus (10) as claimed in any of the preceding claims, **characterized in that** the heat exchange apparatus (30) includes a fluid as heat carrier.

9. The apparatus (10) as claimed in any of the preceding claims, **characterized in that** the apparatus (10) includes a cooler, wherein the cooler is disposed upstream of the inlet (50).

10. The apparatus (10) as claimed in claim 9, **characterized in that** the cooler has a water outlet.

**Revendications**

1. Appareil (10) destiné à séparer le dioxyde de carbone d'un mélange gazeux, dans lequel l'appareil (10) comprend un solide (20) destiné à l'absorption du dioxyde de carbone, dans lequel l'appareil (10) comprend un dispositif d'échange thermique (30) destiné au solide (20), dans lequel l'appareil (10) comprend une enveloppe (40), dans lequel l'enveloppe (40) est pouvant être mise sous vide, dans lequel l'appareil (10) comporte une entrée (50) dans l'enveloppe (40) pour le mélange gazeux, dans lequel l'entrée (50) est disposée au-dessus du solide (20), **l' t caractérisé en ce que** le solide (20) destiné à l'absorption du dioxyde de carbone se présente sous forme de particules, dans lequel l'entrée (50) est conçue pour générer un écoulement radial du mélange gazeux au-dessus du solide (20), dans lequel des éléments conducteurs (110) sont disposés dans l'enveloppe (40) et au niveau de l'entrée (50).

2. L'appareil (10) selon la revendication 1, **caractérisé en ce que** l'enveloppe (40) présente une section transversale circulaire.

3. L'appareil (10) selon l'une quelconque des revendications précédentes, **caractérisé en ce que** le solide (20) est disposé dans une couche d'absorption selon une configuration horizontale, dans lequel la couche d'absorption comporte un élément de limite supérieur (70) à l'extrémité supérieure et un élément de limite inférieur (80) à l'extrémité inférieure, dans lequel l'élément de limite supérieur (70) et l'élément de limite inférieur (80) sont chacun formés par une grille ou une plaque perforée, les ouvertures de la grille ou de la plaque perforée étant plus petites que la taille des particules du solide (20).

4. L'appareil (10) selon la revendication 3, **caractérisé en ce que** l'élément de limite supérieur (70) comporte des structures verticales (90), lesdites structures verticales s'étendant dans le solide (20).

5. L'appareil (10) selon l'une quelconque des revendications précédentes, **caractérisé en ce que** l'appareil (10) comporte une pompe pour évacuer l'enveloppe (40), dans lequel la pompe est une pompe à anneau d'eau.

6. Dispositif (10) selon l'une quelconque des revendications précédentes, **caractérisé en ce que** le dispositif (10) comprend un dispositif de transport pneumatique (100) pour le solide (20).

7. L'appareil (10) selon la revendication 6, **caractérisé en ce que** le dispositif de transport pneumatique comporte un tube de raccordement, le tube de raccordement pouvant être vidé pour retirer le solide (20) de l'enveloppe (40), le solide (20) pouvant être introduit dans l'enveloppe (40) avec un flux de gaz à travers le tube de raccordement.

8. L'appareil (10) selon l'une quelconque des revendications précédentes, **caractérisé en ce que** l'appareil d'échange thermique (30) comprend un fluide servant de caloporteur.

9. L'appareil (10) selon l'une quelconque des revendications précédentes, **caractérisé en ce que** l'appareil (10) comprend un refroidisseur, le refroidisseur étant disposé en amont de l'entrée (50).

10. L'appareil (10) selon la revendication 9, **caractérisé en ce que** le refroidisseur comporte une sortie d'eau.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008015150 B4
- DE 102018212898 A1
- DE 69728061 T2
- WO 2017212381 A1
- WO 2014011081 A2
- EP 2963107 A1
- US 5762692 A